# EUROPEAN PATENT APPLICATION

(11) **EP 1 310 222 A2**
(43) Date of publication of application: **14.05.2003**
(21) Application number: 02257644.1
(22) Date of filing: 05.11.2002
(51) Int. Cl.: A61F 9/007

(54) **Drug-releasing trabecular implant for glaucoma treatment**

(30) Priority: 08.11.2001 US 46137
(71) Applicant: Glaukos Corporation, Laguna Hills, CA 92653 (US)
(72) Inventor: Tu, Hosheng, Newport Coast, California 92657 (US); Niksch, Barbara, Laguna Niguel, California 92677 (US); Haffner, David, Mission Viejo, California 92692 (US); Smedley, Gregory T., Irvine, California 92612 (US)
(74) Representative: Hector, Annabel Mary

(57) **Abstract**

A device and method are provided for improved treatment of elevated intraocular pressure due to glaucoma. A trabecular shunting device is adapted for implantation within the trabecular meshwork of an eye such that aqueous humor flows controllably from the anterior chamber of the eye to Schlemm's canal, bypassing the trabecular meshwork. The trabecular shunting device may utilize a quantity of pharmaceuticals effective in treating glaucoma, which are controllably released from the device into cells of the trabecular meshwork and/or Schlemm's canal. Depending upon the specific treatment contemplated, pharmaceuticals may be utilized in conjunction with the trabecular shunting device such that aqueous flow either increases or decreases as desired. Placement of the trabecular shunting device within the eye, and release of a glaucoma medication therefrom, can arrest or slow the progression of glaucoma.

## Description

### Cross Reference to Related Application

This application claims the priority benefit of U.S. Provisional Application No. 60/281,247, entitled "Drug Slow Release for Glaucoma Treatment," filed April 3, 2001, the entirety of which is hereby incorporated by reference.

### Background of the Invention

### Field of the Invention

This invention relates to reducing intraocular pressure within the animal eye. More particularly, this invention relates to a treatment of glaucoma wherein aqueous humor is permitted to flow out of an anterior chamber of the eye through a surgically implanted pathway. Furthermore, this invention relates to a direct delivery of pharmaceuticals to ocular tissue through an implant.

### Description of the Related Art

As is well known in the art, a human eye is a specialized sensory organ capable of light reception and is able to receive visual images. Aqueous humor is a transparent liquid that fills the region between the cornea, at the front of the eye, and the lens. A trabecular meshwork, located in an anterior chamber angle formed between the iris and the cornea, serves as a drainage channel for aqueous humor from the anterior chamber, which maintains a balanced pressure within the anterior chamber of the eye.

About two percent of people in the United States have glaucoma. Glaucoma is a group of eye diseases encompassing a broad spectrum of clinical presentations, etiologies, and treatment modalities. Glaucoma causes pathological changes in the optic nerve, visible on the optic disk, and it causes corresponding visual field loss, resulting in blindness if untreated. Lowering intraocular pressure is the major treatment goal in all glaucomas.

In glaucomas associated with an elevation in eye pressure (intraocular hypertension), the source of resistance to outflow is mainly in the trabecular meshwork. The tissue of the trabecular meshwork allows the aqueous humor (hereinafter referred to as "aqueous") to enter Schlemm's canal, which then empties into aqueous collector channels in the posterior wall of Schlemm's canal and then into aqueous veins, which form the episcleral venous system. Aqueous is continuously secreted by a ciliary body around the lens, so there is a constant flow of aqueous from the ciliary body to the anterior chamber of the eye. Pressure within the eye is determined by a balance between the production of aqueous and its exit through the trabecular meshwork (major route) and uveal scleral outflow (minor route). The portion of the trabecular meshwork adjacent to Schlemm's canal (the juxtacanilicular meshwork) causes most of the resistance to aqueous outflow.

Glaucoma is broadly classified into two categories: closed-angle glaucoma, also known as angle closure glaucoma, and open-angle glaucoma. Closed-angle glaucoma is caused by closure of the anterior chamber angle by contact between the iris and the inner surface of the trabecular meshwork. Closure of this anatomical angle prevents normal drainage of aqueous from the anterior chamber of the eye. Open-angle glaucoma is any glaucoma in which the exit of aqueous through the trabecular meshwork is diminished while the angle of the anterior chamber remains open. For most cases of open-angle glaucoma, the exact cause of diminished filtration is unknown. Primary open-angle glaucoma is the most common of the glaucomas, and is often asymptomatic in the early to moderately advanced stages of glaucoma. Patients may suffer substantial, irreversible vision loss prior to diagnosis and treatment. However, there are secondary open-angle glaucomas which may include edema or swelling of the trabecular spaces (e.g., from corticosteroid use), abnormal pigment dispersion, or diseases such as hyperthyroidism that produce vascular congestion.

All current therapies for glaucoma are directed toward decreasing intraocular pressure. Currently recognized categories of drug therapy for glaucoma include: (1) Miotics (e.g., pilocarpine, carbachol, and acetylcholinesterase inhibitors), (2) Sympathomimetics (e.g., epinephrine and dipivalylepinephxine), (3) Beta-blockers (e.g., betaxolol, levobunolol and timolol), (4) Carbonic anhydrase inhibitors (e.g., acetazolamide, methazolamide and ethoxzolamide), and (5) Prostaglandins (e.g., metabolite derivatives of arachindonic acid). Medical therapy includes topical ophthalmic drops or oral medications that reduce the production of aqueous or increase the outflow of aqueous. However, drug therapies for glaucoma are sometimes associated with significant side effects. The most frequent and perhaps most serious drawback to drug therapy is that patients, especially the elderly, often fail to correctly self-medicate. Such patients forget to take their medication at the appropriate times or else administer eye drops improperly, resulting in under- or over-dosing. Because the effects of glaucoma are irreversible, when patients dose improperly, allowing ocular concentrations to drop below appropriate therapeutic levels, further permanent damage to vision occurs. Furthermore, current drug therapies are targeted to be deposited directly into the ciliary body where the aqueous is produced. And, current therapies do not provide for a continuous slow-release of the drug. When drug therapy fails, surgical therapy is pursued.

Surgical therapy for open-angle glaucoma consists of laser trabeculoplasty, trabeculectomy, and implantation of aqueous shunts after failure of trabeculectomy or if trabeculectomy is unlikely to succeed. Trabeculectomy is a major surgery that is widely used and is augmented with topically applied anticancer drugs, such as 5-flurouracil or mitomycin-C to decrease scarring and increase the likelihood of surgical success.

Approximately 100,000 trabeculectomies are performed on Medicare-age patients per year in the United States. This number would likely increase if ocular morbidity associated with trabeculectomy could be decreased. The current morbidity associated with trabeculectomy consists of failure (10-15%); infection (a life long risk of 2-5%); choroidal hemorrhage, a severe internal hemorrhage from low intraocular pressure, resulting in visual loss (1%); cataract formation; and hypotony maculopathy (potentially reversible visual loss from low intraocular pressure). For these reasons, surgeons have tried for decades to develop a workable surgery for the trabecular meshwork.

The surgical techniques that have been tried and practiced are goniotomy/trabeculotomy and other mechanical disruptions of the trabecular meshwork, such as trabeculopuncture, goniophotoablation, laser trabecular ablation, and goniocurretage. These are all major operations and are briefly described below.

Goniotomy and trabeculotomy are simple and directed techniques of microsurgical dissection with mechanical disruption of the trabecular meshwork. These initially had early favorable responses in the treatment of open-angle glaucoma. However, long-term review of surgical results showed only limited success in adults. In retrospect, these procedures probably failed due to cellular repair and fibrosis mechanisms and a process of "filling in." Filling in is a detrimental effect of collapsing and closing in of the created opening in the trabecular meshwork. Once the created openings close, the pressure builds back up and the surgery fails.

Q-switched Neodynium (Nd) YAG lasers also have been investigated as an optically invasive trabeculopuncture technique for creating full-thickness holes in trabecular meshwork. However, the relatively small hole created by this trabeculopuncture technique exhibits a filling-in effect and fails.

Goniophotoablation is disclosed by Berlin in U.S. Pat. No. 4,846,172 and involves the use of an excimer laser to treat glaucoma by ablating the trabecular meshwork. This method did not succeed in a clinical trial. Hill et al. used an Erbium YAG laser to create full-thickness holes through trabecular meshwork (Hill et al., Lasers in Surgery and Medicine 11:341346, 1991). This laser trabecular ablation technique was investigated in a primate model and a limited human clinical trial at the University of California, Irvine. Although ocular morbidity was zero in both trials, success rates did not warrant further human trials. Failure was again from filling in of surgically created defects in the trabecular meshwork by repair mechanisms. Neither of these is a viable surgical technique for the treatment of glaucoma.

Goniocurretage is an "ab interno" (from the inside), mechanically disruptive technique that uses an instrument similar to a cyclodialysis spatula with a microcurrette at the tip. Initial results were similar to trabeculotomy: it failed due to repair mechanisms and a process of filling in.

Although trabeculectomy is the most commonly performed filtering surgery, viscocanulostomy (VC) and non penetrating trabeculectomy (NPT) are two new variations of filtering surgery. These are "ab externo" (from the outside), major ocular procedures in which Schlemm's canal is surgically exposed by making a large and very deep scleral flap. In the VC procedure, Schlemm's canal is cannulated and viscoelastic substance injected (which dilates Schlemm's canal and the aqueous collector channels). In the NPT procedure, the inner wall of Schlemm's canal is stripped off after surgically exposing the canal.

Trabeculectomy, VC, and NPT involve the formation of an opening or hole under the conjunctiva and scleral flap into the anterior chamber, such that aqueous is drained onto the surface of the eye or into the tissues located within the lateral wall of the eye. These surgical operations are major procedures with significant ocular morbidity. When trabeculectomy, VC, and NPT are thought to have a low chance for success, a number of implantable drainage devices have been used to ensure that the desired filtration and outflow of aqueous through the surgical opening will continue. The risk of placing a glaucoma drainage device also includes hemorrhage, infection, and diplopia (double vision).

Examples of implantable shunts and surgical methods for maintaining an opening for the release of aqueous from the anterior chamber of the eye to the sclera or space beneath the conjunctiva have been disclosed in, for example, Hsia et al., U.S. Patent No. 6,059,772 and Baerveldt, U.S. Patent No. 6,050,970.

All of the above embodiments and variations thereof have numerous disadvantages and moderate success rates. They involve substantial trauma to the eye and require great surgical skill in creating a hole through the full thickness of the sclera into the subconjunctival space. The procedures are generally performed in an operating room and involve a prolonged recovery time for vision. The complications of existing filtration surgery have prompted ophthalmic surgeons to find other approaches to lowering intraocular pressure.

Because the trabecular meshwork and juxtacanilicular tissue together provide the majority of resistance to the outflow of aqueous, they are logical targets for surgical removal in the treatment of open-angle glaucoma. In addition, minimal amounts of tissue need be altered and existing physiologic outflow pathways can be utilized.

As reported in Arch. Ophthalm. (2000) 118:412, glaucoma remains a leading cause of blindness, and filtration surgery remains an effective, important option in controlling glaucoma. However, modifying existing filtering surgery techniques in any profound way to increase their effectiveness appears to have reached a dead end. The article further states that the time has come to search for new surgical approaches that may provide better and safer care for patients with glaucoma.

What is needed, therefore, is an extended, site specific treatment method for glaucoma that is faster, safer, and less expensive than currently available modalities.

### Summary of the Invention

A device and method are provided for improved treatment of intraocular pressure due to glaucoma. A trabecular shunting device is adapted for implantation within a trabecular meshwork of an eye such that aqueous humor flows controllably from an anterior chamber of the eye to Schlemm's canal, bypassing the trabecular meshwork. The trabecular shunting device comprises a quantity of pharmaceuticals effective in treating glaucoma, which are controllably released from the device into cells of the trabecular meshwork and/or Schlemm's canal. Depending upon the specific treatment contemplated, pharmaceuticals may be utilized in conjunction with the trabecular shunting device such that aqueous flow either increases or decreases as desired. Placement of the trabecular shunting device within the eye and incorporation, and eventual release, of a proven pharmaceutical glaucoma therapy will reduce, inhibit or slow the effects of glaucoma.

One aspect of the invention provides a trabecular shunting device that is implantable within an eye. The device comprises an inlet section containing at least one lumen, a flow-restricting member within the lumen that is configured to prevent at least one component of blood from passing through the flow-restricting member, an outlet section having a first outlet end and a second, opposite outlet end. The outlet section has at least one lumen which opens to at least one of the first and second outlet ends. The device further comprises a middle section having at least one lumen. The middle section is fixedly attached to the outlet section between the first and second outlet ends, and the lumen is in fluid communication with the lumen of the outlet section. The middle section is fixedly attached to the inlet section and the lumen within the middle section in fluid communication with the lumen of the inlet section. The device is configured to permit fluid entering the lumen of the inlet section to pass through the flow-restricting member, enter the lumen of the middle section, pass into the lumen of the outlet section, and then exit the outlet section through at least one of the first and second outlet ends.

Another aspect of the invention provides a method of treating glaucoma. The method comprises providing at least one pharmaceutical substance incorporated into a trabecular shunting device, implanting the trabecular shunting device within a trabecular meshwork of an eye such that a first end of the trabecular shunt is positioned in an anterior chamber of the eye while a second end is positioned in a Schlemm's canal, and allowing the shunting device to release a quantity of the pharmaceutical substance into the eye. The first and second ends of the trabecular shunting device establish a fluid communication between the anterior chamber and the Schlemm's canal.

In another aspect of the invention, a method of regulating aqueous humor outflow within an eye is provided. The method comprises creating an incision in a trabecular meshwork of the eye, wherein the incision is substantially parallel with a circumference of a limbus of the eye, inserting an outlet section of a trabecular shunting device through the incision into Schlemm's canal such that the outlet section resides within Schlemm's canal while an inlet section of the trabecular shunting device resides in the anterior chamber, and initiating an outflow of aqueous humor from the anterior chamber through the trabecular shunting device into Schlemm's canal.

Still another aspect of the invention provides a method of regulating intraocular pressure within an eye. The method comprises making an incision passing into a trabecular meshwork of the eye, wherein the incision is oriented lengthwise substantially parallel with a circumference of a limbus. The incision establishes a fluid communication between an anterior chamber and Schlemm's canal of the eye. The method further comprises implanting a trabecular shunting device through the incision such that an outlet section of the trabecular shunting device resides within Schlemm's canal and an inlet section of the trabecular shunting device resides within the anterior chamber. The method still further comprises establishing a fluid transfer from the anterior chamber through the trabecular shunting device into Schlemm's canal.

Another aspect of the invention provides an apparatus for implanting a trabecular shunting device within an eye. The apparatus comprises a syringe portion and a cannula portion that has proximal and distal ends. The proximal end of the cannula portion is attached to the syringe portion. The cannula portion further comprises a first lumen and at least one irrigating hole disposed between the proximal and distal ends of the cannula portion. The irrigating hole is in fluid communication with the lumen. The apparatus further includes a holder comprising a second lumen for holding the trabecular shunting device. A distal end of the second lumen opens to the distal end of the cannula portion, and a proximal end of the second lumen is separated from the first lumen of the cannula portion. The holder holds the trabecular shunting device during implantation of the device within the eye, and the holder releases the trabecular shunting device when a practitioner activates deployment of the device.

Another aspect of the invention provides a method of implanting a trabecular shunting device within an eye. The method comprises creating a first incision in a cornea on a first side of the eye, wherein the first incision passes through the cornea into an anterior chamber of the eye. The method further comprises passing an incising device through the first incision and moving a distal end of the incising device across the anterior chamber to a trabecular meshwork residing on a second side of the eye, and using the incising device to create a second incision. The second incision is in the trabecular meshwork, passing from the anterior chamber through the trabecular meshwork into a Schlemm's canal. The method further comprises inserting the trabecular shunting device into a distal space of a delivery applicator. The delivery applicator comprises a cannula portion having a distal end and a proximal end attached to a syringe portion. The cannula portion has at least one lumen and at least one irrigating hole disposed between proximal and distal ends of the cannula portion. The irrigating hole is in fluid communication with the lumen. The distal space comprises a holder that holds the trabecular shunting device during delivery and releases the trabecular shunting device when a practitioner activates deployment of the device. The method further comprises advancing the cannula portion and the trabecular shunting device through the first incision, across the anterior chamber and into the second incision, wherein an outlet section of the trabecular shunting device is implanted into Schlemm's canal while an inlet section of the trabecular shunting device remains in fluid communication with the anterior chamber. The method still further comprises releasing the trabecular shunting device from the holder of the delivery applicator.

### Brief Description of the Drawings

**FIGURE 1** is a coronal, cross-sectional view of an eye.

**FIGURE 2** is an enlarged cross-sectional view of an anterior chamber angle of the eye of **FIGURE 1**.

**FIGURE 3** is an oblique elevation view of one embodiment of a trabecular shunting device.

**FIGURE 4** is an oblique elevation view of another embodiment of a trabecular shunting device.

**FIGURE 5A** is an oblique elevation view of placement of one end of a trabecular shunting device through a trabecular meshwork.

**FIGURE 5B** is an oblique elevation view of placement of one end of a trabecular shunting device through a trabecular meshwork, wherein the trabecular shunting device is passed over a guidewire.

**FIGURE 6** is an oblique elevation view of a preferred implantation of a trabecular shunting device through a trabecular meshwork.

**FIGURE 7** is an enlarged, cross-sectional view of a preferred method of implanting a trabecular shunting device within an eye.

**FIGURE 8** is a perspective view of an anterior chamber angle of an eye, illustrating a trabecular shunting device positioned within a trabecular meshwork.

**FIGURE 9** is a close-up, cut-away view of an inlet section of the trabecular shunting device of **FIGURES 3** and **4**, illustrating a flow-restricting member retained within a lumen of the trabecular shunting device.

### Detailed Description of the Preferred Embodiments

The preferred embodiments of the present invention described below relate particularly to surgical and therapeutic treatment of glaucoma through reduction of intraocular pressure. While the description sets forth various embodiment specific details, it will be appreciated that the description is illustrative only and should not to be construed in any way as limiting the invention. Furthermore, various applications of the invention, and modifications thereto, which may occur to those who are skilled in the art, are also encompassed by the general concepts described below.

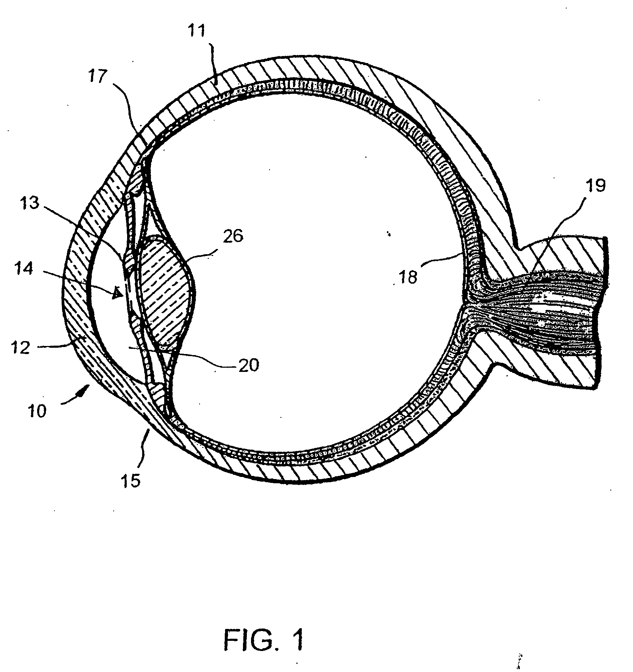

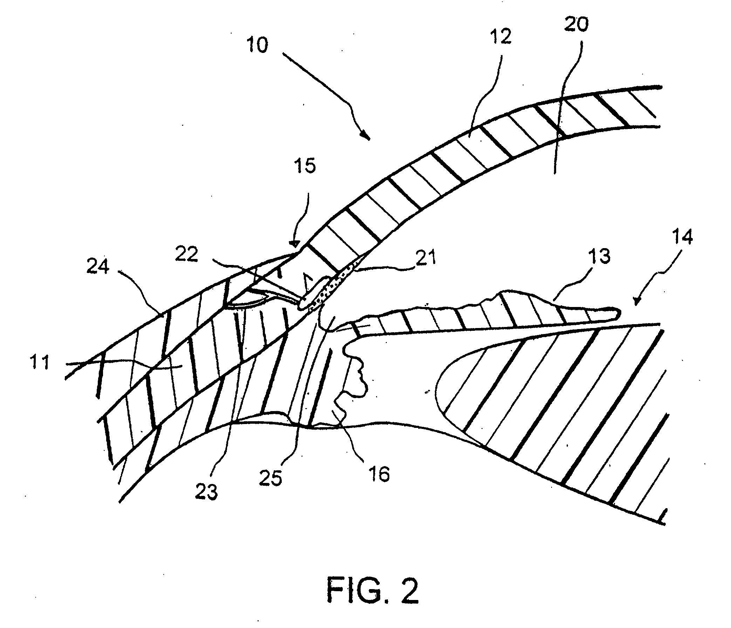

**FIGURE 1** is a cross-sectional view of an eye 10, while **FIGURE 2** is a close-up view showing the relative anatomical locations of a trabecular meshwork 21, an anterior chamber 20, and a Schlemm's canal 22. A sclera 11 is a thick collagenous tissue which covers the entire eye 10 except a portion which is covered by a cornea 12. The cornea 12 is a thin transparent tissue that focuses and transmits light into the eye and through a pupil 14, which is a circular hole in the center of an iris 13 (colored portion of the eye). The cornea 12 merges into the sclera 11 at a juncture referred to as a limbus 15. A ciliary body 16 extends along the interior of the sclera 11 and is coextensive with a choroid 17. The choroid 17 is a vascular layer of the eye 10, located between the sclera 1I and a retina 18. An optic nerve 19 transmits visual information to the brain and is the anatomic structure that is progressively destroyed by glaucoma.

The anterior chamber 20 of the eye 10, which is bound anteriorly by the cornea 12 and posteriorly by the iris 13 and a lens 26, is filled with aqueous humor (hereinafter referred to as "aqueous"). Aqueous is produced primarily by the ciliary body 16, then moves anteriorly through the pupil 14 and reaches an anterior chamber angle 25, formed between the iris 13 and the cornea 12. In a normal eye, aqueous is removed from the anterior chamber 20 through the trabecular meshwork 21. Aqueous passes through the trabecular meshwork 21 into Schlemm's canal 22 and thereafter through a plurality of aqueous veins 23, which merge with blood-carrying veins, and into systemic venous circulation. Intraocular pressure is maintained by an intricate balance between secretion and outflow of aqueous in the manner described above. Glaucoma is, in most cases, characterized by an excessive buildup of aqueous in the anterior chamber 20 which leads to an increase in intraocular pressure. Fluids are relatively incompressible, and thus intraocular pressure is distributed relatively uniformly throughout the eye 10.

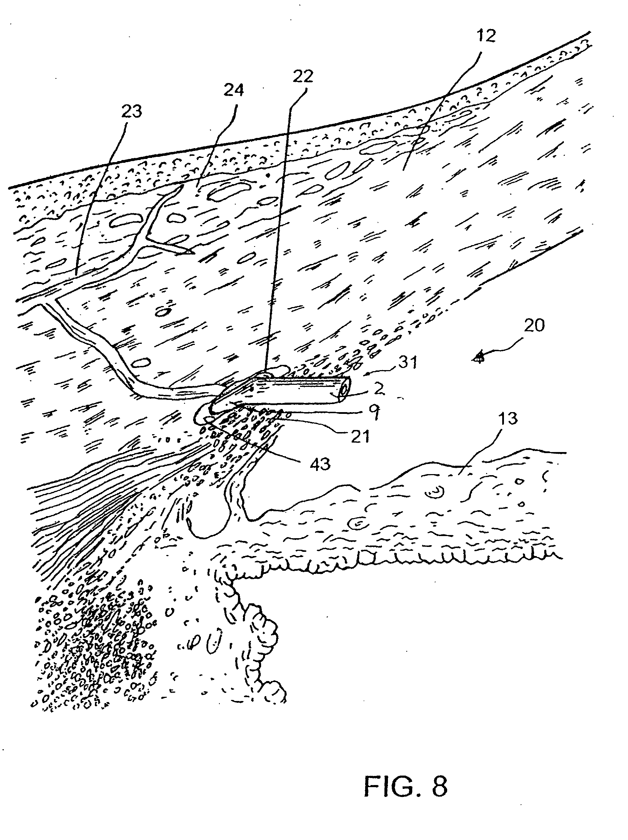

As shown in **FIGURE 2**, the trabecular meshwork 21 is adjacent a small portion of the sclera 11. Exterior to the sclera 11 is a conjunctiva 24. Traditional procedures that create a hole or opening for implanting a device through the tissues of the conjunctiva 24 and sclera 11 involve extensive surgery, as compared to surgery for implanting a device, as described herein, which ultimately resides entirely within the confines of the sclera 1I and cornea 12. **FIGURE 8** generally illustrates the use of one embodiment of a trabecular shunting device 31 for establishing an outflow pathway, passing through the trabecular meshwork 21, which is discussed in greater detail below.

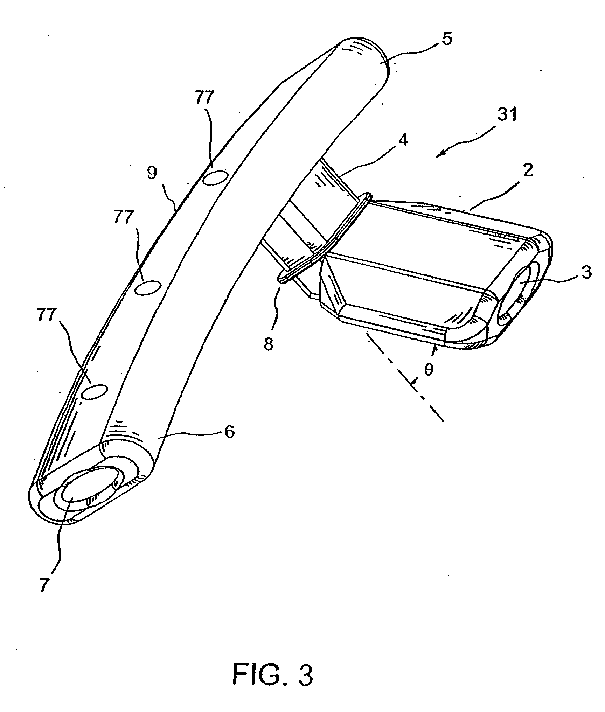

**FIGURE 3** illustrates a preferred embodiment of a trabecular shunting device 31 which facilitates the outflow of aqueous from the anterior chamber 20 into Schlemm's canal 22, and subsequently into the aqueous collectors and the aqueous veins so that intraocular pressure is reduced. In the illustrated embodiment, the trabecular shunting device 31 comprises an inlet section 2, having an inlet opening 3, a middle section 4, and an outlet section 9. The middle section 4 may be an extension of, or may be coextensive with, the inlet section 2. The outlet section 9 is preferably somewhat flexible to facilitate positioning of the outlet section 9 within an outflow pathway of the eye 10. The outlet section 9 is preferably substantially perpendicular to the middle section 4. "Substantially perpendicular," as used herein, is defined as subtending an angle between longitudinal axes of the sections 4, 9 ranging between about 30 degrees and about 150 degrees. The device 31 further comprises at least one lumen 7 within sections 4 and 9 which is in fluid communication with the inlet opening 3 of section 2, thereby facilitating transfer of aqueous through the device 31.

The outlet section 9 preferably has a first outlet end 6 and a second, opposite outlet end 5. The lumen 7 within the outlet section 9 opens to at least one of the outlet ends 5,6. Furthermore, the outlet section 9 may have a plurality of side openings 77, each of which is in fluid communication with the lumen 7, for transmission of aqueous. The middle section 4 is connected to or coextensive with the outlet section 9 and is disposed between the first outlet end 6 and the second outlet end 5. In a preferred embodiment, the outlet section 9 is curved around a point, or curve center, and the middle section 4 extends substantially along a plane that contains the curve center. In this embodiment, the outlet section 9 has a radius of curvature ranging between about 4 mm and about 10 mm.

As will be apparent to a person skilled in the art, the lumen 7 and the remaining body of the outlet section 9 may have a cross-sectional shape that is oval, circular, or other appropriate shape. The cross-sectional shapes of the lumen 7 and the outlet section 9 preferably conform to the shape of the outflow pathway into which the outlet section 9 is placed. The opening of the lumen 7 of the outlet ends 5,6 may be ovoid in shape to match the contour of Schlemm's canal 22. Further, an outer contour of the outlet section 9 may be elliptical (e.g., ovoid) in shape to match the contour of Schlemm's canal 22. This serves to minimize rotational movement of the outlet section 9 within Schlemm's canal 22, and thereby stabilizes the inlet section 2 with respect to the iris and cornea.

A circumferential ridge 8 is provided at the junction of the inlet section 2 and the middle section 4 to facilitate stabilization of the device 31 once implanted within the eye 10. Preferably, the middle section 4 has a length (between the ridge 8 and the outlet section 9) that is roughly equal to a thickness of the trabecular meshwork 21, which typically ranges between about 100 µm and about 300 µm. In addition, the outlet section 9 may advantageously be formed with a protuberance or spur projecting therefrom so as to further stabilize the device 31 within the eye 10 without undue suturing.

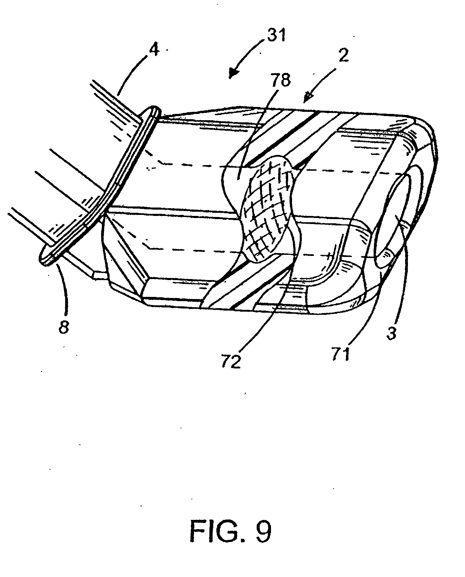

**FIGURE 9** is a close-up view of the inlet section 2 of the trabecular shunting device 31, illustrating a flow-restricting member 72 which is tightly retained within a lumen 78. The flow-restricting member 72 is shown located close to an inlet side 71 of the inlet section 2. The flow-restricting member 72 serves to selectively restrict at least one component in blood from moving retrograde, i.e., from the outlet section 9 into the anterior chamber 20 of the eye 10. Alternatively, the flow-restricting member 72 may be situated in any location within the device 31 such that blood flow is restricted from retrograde motion. The flow-restricting member 72 may, in other embodiments, be a filter made of a material selected from the following filter materials: expanded polytetrafluoroethylene, cellulose, ceramic, glass, Nylon, plastic, and fluorinated material such as polyvinylidene fluoride ("PVDF") (trade name: Kynar, by DuPont).

The trabecular shunting device 31 may be made by molding, thermo-forming, or other micro-machining techniques. The trabecular shunting device 31 preferably comprises a biocompatible material such that inflammation arising due to irritation between the outer surface of the device 31 and the surrounding tissue is minimized. Biocompatible materials which may be used for the device 31 preferably include, but are not limited to, titanium, medical grade silicone, e.g., Silastic™, available from Dow Coming Corporation of Midland, Michigan; and polyurethane, e.g., Pellethane™, also available from Dow Corning Corporation. In other embodiments, the device 31 may comprise other types of biocompatible material, such as, by way of example, polyvinyl alcohol, polyvinyl pyrolidone, collagen, heparinized collagen, polytetrafluoroethylene, expanded polytetrafluoroethylene, fluorinated polymer, fluorinated elastomer, flexible fused silica, polyolefin, polyester, polysilicon, and/or a mixture of the aforementioned biocompatible materials, and the like. In still other embodiments, composite biocompatible material may be used, wherein a surface material may be used in addition to one or more of the aforementioned materials. For example, such a surface material may include polytetrafluoroethylene (PTFE) (such as Teflon™), polyimide, hydrogel, heparin, therapeutic drugs (such as beta-adrenergic antagonists and other anti-glaucoma drugs, or antibiotics), and the like.

As is well known in the art, a device coated or loaded with a slow-release substance can have prolonged effects on local tissue surrounding the device. The slow-release delivery can be designed such that an effective amount of substance is released over a desired duration. "Substance", as used herein, is defined as any therapeutic or active drug that can stop, mitigate, slow-down or reverse undesired disease processes.

In one embodiment, the device 31 may be made of a biodegradable (also including bioerodible) material admixed with a substance for substance slow-release into ocular tissues. In another embodiment, polymer films may function as substance containing release devices whereby the polymer films may be coupled or secured to the device 31. The polymer films may be designed to permit the controlled release of the substance at a chosen rate and for a selected duration, which may also be episodic or periodic. Such polymer films may be synthesized such that the substance is bound to the surface or resides within a pore in the film so that the substance is relatively protected from enzymatic attack. The polymer films may also be modified to alter their hydrophilicity, hydrophobicity and vulnerability to platelet adhesion and enzymatic attack.

Furthermore, the film may be coupled (locally or remotely) to a power source such that when substance delivery is desired, a brief pulse of current is provided to alter the potential on the film to cause the release of a particular amount of the substance for a chosen duration. Application of current causes release of a substance from the surface of the film or from an interior location in the film such as within a pore. The rate of substance delivery is altered depending on the degree of substance loading on the film, the voltage applied to the film, and by modifying the chemical synthesis of substance delivery polymer film.

The power-activated substance delivery polymer film may be designed to be activated by an electromagnetic field, such as, by way of example, NMR, MRI, or short range RF transmission (such as Bluetooth). In addition, ultrasound can be used to cause a release of a particular amount of substance for a chosen duration. This is particularly applicable to a substance coated device or a device made of a substrate containing the desired substance.

The device 31 may be used for a direct release of pharmaceutical preparations into ocular tissues. As discussed above, the pharmaceuticals may be compounded within the device 31 or form a coating on the device 31. Any known drug therapy for glaucoma may be utilized, including but not limited to, the following:
U.S. Pat. No. 6,274,138, issued August 14, 2001 and U.S. Pat. No. 6,231,853, issued May 15, 2001, the entire contents of both of which are incorporated herein by reference, disclose the function of mitochondria and toxic substances synthesized as a metabolic byproduct within mitochondria of cells. Perry and associates (Perry HD et al. "Topical cyclosporin A in the management of postkeratoplasty glaucoma" Cornea 16:284-288, 1997) report that topical cyclosporin-A has been shown to reduce post-surgical increases in intraocular pressure. It is proposed that such compounds with known effects on mitochondrial stability might be effective in treating trabecular meshwork. An antagonistic drug to neutralize the toxic byproduct or a stabilizing drug to effect mitochondrial stability is believed able to restore the mitochondria function and subsequently mitigate the dysfunction of the trabecular meshwork.
U.S. Patent Application No. 6,201,001, issued March 13, 2001, the entire contents of which are incorporated herein by reference, discloses Imidazole antiproliferative agents useful for neovascular glaucoma;
U.S. Patent Application No. 6,228,873, issued May 8, 2001, the entire contents of which are incorporated herein by reference, discloses a new class of compounds that inhibit function of sodium chloride transport in the thick ascending limb of the loop of Henle, wherein the preferred compounds useful are furosemide, piretanide, benzmetanide, bumetanide, torasernide and derivatives thereof.
U.S. Patent Application No. 6,194,415, issued February 27, 2001, the entire contents of which are incorporated herein by reference, discloses a method of using quinoxoalines (2-imidazolin-2-ylamino) in treating neural injuries (e.g. glaucomatous nerve damage);
U.S. Patent Application No. 6,060,463, issued May 9, 2000, and U.S. Patent Application No. 5,869,468, issued February 9, 1999, the entire contents of which are incorporated herein by reference, disclose treatment of conditions of abnormally increased intraocular pressure by administration of phosphonylmethoxyalkyl nucleotide analogs and related nucleotide analogs;
U.S. Patent Application No. 5,925,342, issued July 20, 1999, the entire contents of which are incorporated herein by reference, discloses a method for reducing intraocular pressure by administration of potassium channel blockers;
U.S. Patent Application No. 5,814,620, issued September 29, 1998, the entire contents of which are incorporated herein by reference, discloses a method of reducing neovascularization and of treating various disorders associated with neovascularization. These methods include administering to a tissue or subject a synthetic oligonucleotide;
U.S. Patent Application No. 5,767,079, issued June 16, 1998, the entire contents of which are incorporated herein by reference, discloses a method for treatment of ophthalmic disorders by applying an effective amount of Transforming Growth Factor-Beta (TGF-beta) to the affected region;
U.S. Patent Application No. 5,663,205, issued September 2, 1997, the entire contents of which are incorporated herein by reference, discloses a pharmaceutical composition for use in glaucoma treatment which contains an active ingredient 5-[1-hydroxy-2-[2-(2-methoxyphenoxyl)ethylamino]ethyl]-2-methylbenzenesulfonamide. This agent is free from side effects, and stable and has an excellent intraocular pressure reducing activity at its low concentrations, thus being useful as a pharmaceutical composition for use in glaucoma treatment;
U.S. Patent Application No. 5,652,236, issued July 29, 1997, the entire contents of which are incorporated herein by reference, discloses pharmaceutical compositions and a method for treating glaucoma and/or ocular hypertension in the mammalian eye by administering thereto a pharmaceutical composition which contains as the active ingredient one or more compounds having guanylate cyclase inhibition activity. Examples of guanylate cyclase inhibitors utilized in the pharmaceutical composition and method of treatment are methylene blue, butylated hydroxyanisole and N-methylhydroxylamine;
U.S. Patent Application No. 5,547,993, issued August 20, 1996, the entire contents of which are incorporated herein by reference, discloses that 2-(4methylaminobutoxy) diphenylmethane or a hydrate or pharmaceutically acceptable salt thereof have been found useful for treating glaucoma;
U.S. Patent Application No. 5,502,052, issued March 26, 1996, the entire contents of which are incorporated herein by reference, discloses use of a combination of apraclonidine and timolol to control intraocular pressure. The compositions contain a combination of an alpha-2 agonist (e.g., para-amino clonidine) and a beta blocker (e.g., betaxolol);
U.S. Patent Application No. 6,184,250, issued February 6, 2001, the entire contents of which are incorporated herein by reference, discloses use of cloprostenol and fluprostenol analogues to treat glaucoma and ocular hypertension. The method comprises topically administering to an affected eye a composition comprising a therapeutically effective amount of a combination of a first compound selected from the group consisting of beta-blockers, carbonic anhydrase inhibitors, adrenergic agonists, and cholinergic agonists; together with a second compound;
U.S. Patent Application No. 6,159,458, issued December 12, 2000, the entire contents of which are incorporated herein by reference, discloses an ophthalmic composition that provides sustained release of a water soluble medicament formed by comprising a crosslinked carboxy-containing polymer, a medicament, a sugar and water;
U.S. Patent Application No. 6,110,912, issued August 29, 2000, the entire contents of which are incorporated herein by reference, discloses methods for the treatment of glaucoma by administering an ophthalmic preparation comprising an effective amount of a non-corneotoxic serine-threonine kinase inhibitor, thereby enhancing aqueous outflow in the eye and treatment of the glaucoma. In some embodiments, the method of administration is topical, whereas it is intracameral in other embodiments. In still further embodiments, the method of administration is intracanalicular;
U.S. Patent Application No. 6,177,427, issued January 23, 2001, the entire contents of which are incorporated herein by reference, discloses compositions of non-steroidal glucocorticoid antagonists for treating glaucoma or ocular hypertension; and
U.S. Patent Application No. 5,952,378, issued September 14,1999, the entire contents of which are incorporated herein by reference, discloses the use of prostaglandins for enhancing the delivery of drugs through the uveoscleral route to the optic nerve head for treatment of glaucoma or other diseases of the optic nerve as well as surrounding tissue. The method for enhancing the delivery to the optic nerve head comprises contacting a therapeutically effective amount of a composition containing one or more prostaglandins and one or more drug substances with the eye at certain intervals.

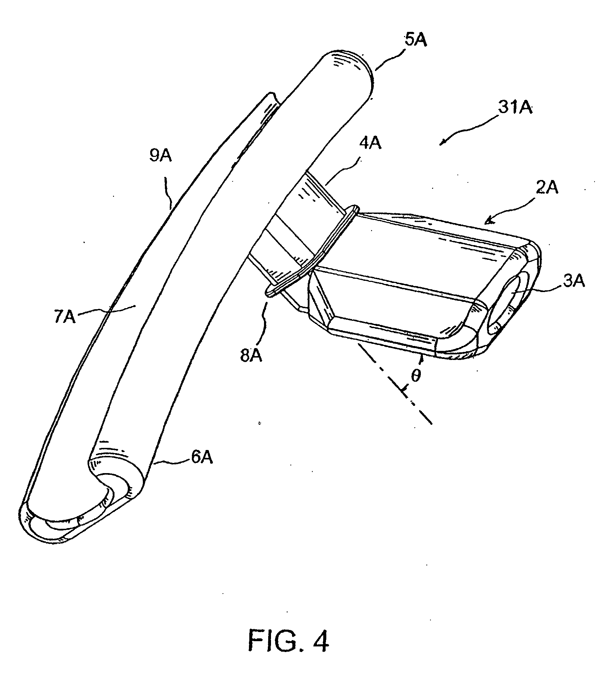

**FIGURE 4** illustrates another embodiment of a trabecular shunting device 31A which facilitates the outflow of aqueous from the anterior chamber 20 into Schlemm's canal 22, and subsequently into the aqueous collectors and the aqueous veins so that intraocular pressure is reduced. The device 31A comprises an inlet section 2A, a middle section 4A, and an outlet section 9A. The device 31A further comprises at least one lumen 3A traversing the sections 2A, 4A; 9A and providing fluid communication therebetween. The lumen 3A facilitates the transfer of aqueous from the inlet section 2A through the device 31A. The outlet section 9A is preferably curved, and may also be somewhat flexible, to facilitate positioning of the outlet section 9A within an existing outflow pathway of the eye 10. The outlet section 9A further comprises an elongate trough 7A for transmitting, or venting, aqueous. The elongate trough 7A is connected to and in fluid communication with the lumen 3A within the trabecular shunting device 31A.

A circumferential ridge 8A is provided at the junction of the inlet section 2A and the middle section 4A to facilitate stabilization of the device 31A once implanted within the eye 10. Preferably, the middle section 4A has a length (between the ridge 8A and the outlet section 9A) that is roughly equal to the thickness of the trabecular meshwork 21, which typically ranges between about 100 µm and about 300 µm. In addition, the outlet section 9A may advantageously be formed with a protuberance or barb projecting therefrom so as to further stabilize the device 31A within the eye 10 without undue suturing.

As will be appreciated by those of ordinary skill in the art, the devices 31 and 31A may advantageously be practiced with a variety of sizes and shapes without departing from the scope of the invention. Depending upon the distance between the anterior chamber 20 and the drainage vessel (e.g., a vein) contemplated, the devices 31, 31A may have a length ranging from about 0.05 centimeters to over 10 centimeters. Preferably, the devices 31 and 31A have an outside diameter ranging between about 30 µm and about 500 µm, with the lumens 7, 3A having diameters ranging between about 20 µm and about 250 µm, respectively. In addition, the devices 31, 31A may have a plurality of lumens to facilitate transmission of multiple flows of aqueous. The inlet sections 2, 2A have longitudinal axes that form an angle (θ) ranging between about 20 degrees and about 150 degrees relative to the longitudinal axes of the middle sections 4, 4A, respectively. More preferably, the angles between the longitudinal axes of the inlet sections 2, 2A and the middle sections 4, 4A range between about 30 degrees and about 60 degrees, respectively.

One preferred method for increasing aqueous outflow in the eye 10 of a patient, to reduce intraocular pressure therein, comprises bypassing the trabecular meshwork 21. In operation, the middle section 4 of the device 31 is advantageously placed across the trabecular meshwork 21 through a slit or opening. This opening can be created by use a laser, a knife, or other surgical cutting instrument. The opening may advantageously be substantially horizontal, i.e., extending longitudinally in the same direction as the circumference of the limbus 15 (**FIGURE 2**). Other opening directions may also be used, as well. The opening may advantageously be oriented at any angle, relative to the circumference of the limbus 15, that is appropriate for inserting the device 31 through the trabecular meshwork 21 and into Schlemm's canal 22 or other outflow pathway, as will be apparent to those skilled in the art. The middle section 4 may be semi-flexible and/or adjustable in position relative to the inlet section 2 and/or the outlet section 9, further adapting the device 31 for simple and safe glaucoma implantation. Furthermore, the outlet section 9 may be positioned into fluid collection channels of the natural outflow pathways. Such natural outflow pathways include Schlemm's canal 22, aqueous collector channels, aqueous veins, and episcleral veins. The outlet section 9 may be positioned into fluid collection channels up to at least the level of the aqueous veins, with the device inserted in a retrograde or antegrade fashion.

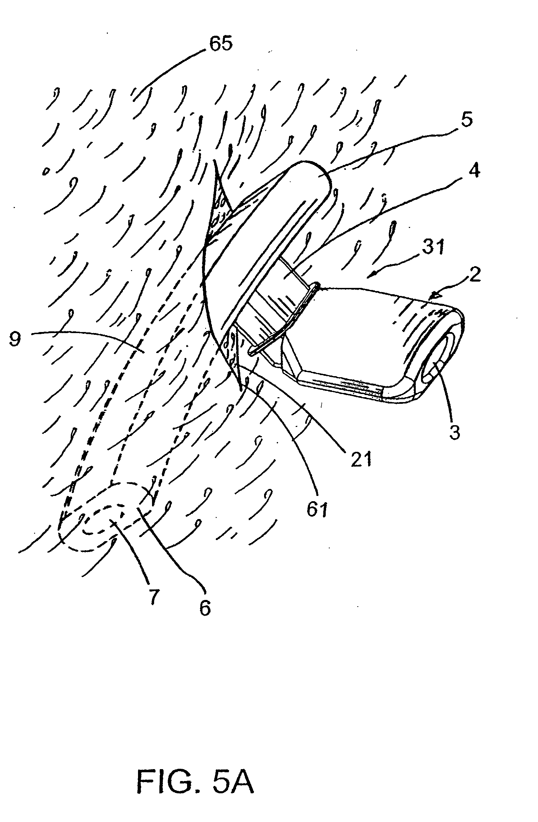

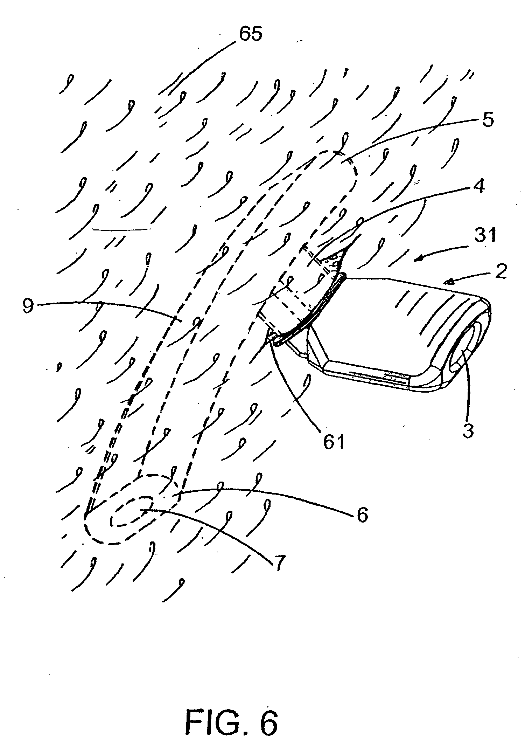

**FIGURE 5A** generally illustrates a step in the implantation of the trabecular shunting device 31 through the trabecular meshwork 21. The outlet section 9 of the device 31 is inserted into an opening 61 in the trabecular meshwork 21. A practitioner may create the opening 61 "ab interno" from the interior surface 65 of the trabecular meshwork 21. The practitioner then advances the first outlet end 6 of the outlet section 9 through the opening 61 into a first side of Schlemm's canal 22 or other suitable outflow pathway within the eye 10. Next, the practitioner advances the second outlet end 5 through the opening 61 and into a second side of Schlemm's canal 22. The advancing of the second outlet end 5 may be facilitated by slightly pushing the second outlet end 5 through the opening 61. **FIGURE 6** generally illustrates a further stage in deployment of the device 31, wherein the entire outlet section 9 of the device 31 is implanted within Schlemm's canal 22, beneath the trabecular meshwork 21. At this stage, the lumen 3 of the implanted device 31 provides an enhanced fluid communication through the trabecular meshwork 21.

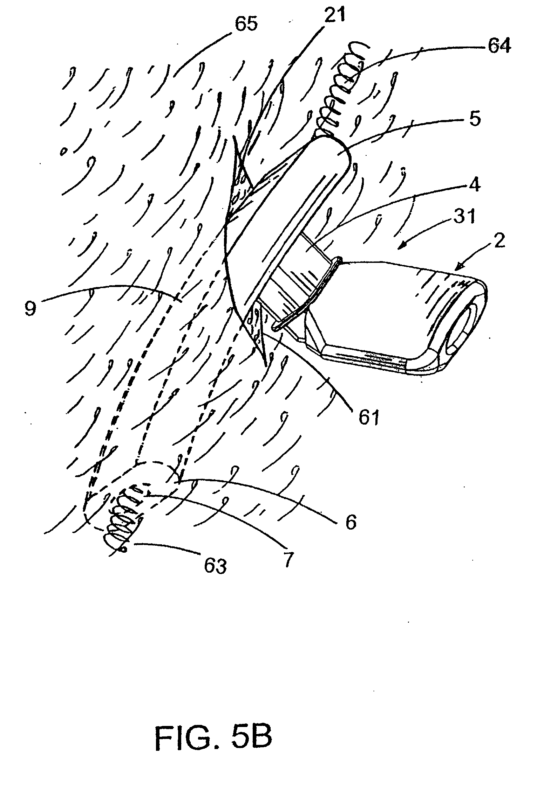

**FIGURE 5B** shows an additional and/or alternate step in the implantation of the trabecular shunting device 31 through the trabecular meshwork 21. The practitioner inserts a distal end 63 of a guidewire 64 through the opening 61 into the first side Schlemm's canal 22. The practitioner then advances the first outlet end 6 of the outlet section 9 into Schlemm's canal 22 by "riding," or advancing, the trabecular shunting device 31 on the guidewire 64. As will be apparent to those skilled in the art, the guidewire 64 will have a shape and size conforming to the shape and size of the lumen 7; and as such, may have an elliptical (e.g., oval) shape, a D-shape, a round shape, or an irregular (asymmetric) shape which is adapted for nonrotatory engagement for the device 31.

Another method for increasing aqueous outflow within the eye 10 of a patient, and thus reduce intraocular pressure therein, comprises: (a) creating an opening in the trabecular meshwork 21, wherein the trabecular meshwork 21 includes a deep side and superficial side; (b) inserting the trabecular shunting device 31 into the opening; and (c) transmitting aqueous through the device 31, to bypass the trabecular meshwork 21, from the deep side to the superficial side of the trabecular meshwork 21. This "transmitting" of aqueous is preferably passive, i.e., aqueous flows out of the anterior chamber 20 due to a pressure gradient between the anterior chamber 20 and the aqueous venous system 23.

Another method for increasing aqueous outflow within the eye 10 of a patient, and thus reduce intraocular pressure therein, comprises a) providing at least one pharmaceutical substance incorporated into a trabecular shunting device at about the middle section of the device; b) implanting the trabecular shunting device within a trabecular meshwork of an eye such that the middle section is configured substantially within the trabecular meshwork, the shunting device having a first end positioned in an anterior chamber of the eye while a second end is positioned inside a Schlemm's canal, wherein the first and the second ends of the trabecular shunting device establish a fluid communication between the anterior chamber and the Schlemm's canal; and c) allowing the middle section of the trabecular shunting device to release a quantity of said pharmaceutical substance into the trabecular meshwork.

It should be understood that the devices 31 and 31A are in now way limited to implantation within only Schlemm's canal 20, as depicted in **FIGURES 5A** and **5B.** Rather, the devices 31 and 31A may advantageously be implanted within and/or used in conjunction with a variety of other natural outflow pathways, or biological tubular structures, as mentioned above. As will be apparent to those of ordinary skill in the art, the devices 31 and 31A may advantageously be used in conjunction with substantially any biological tubular structure without detracting from the scope of the invention.

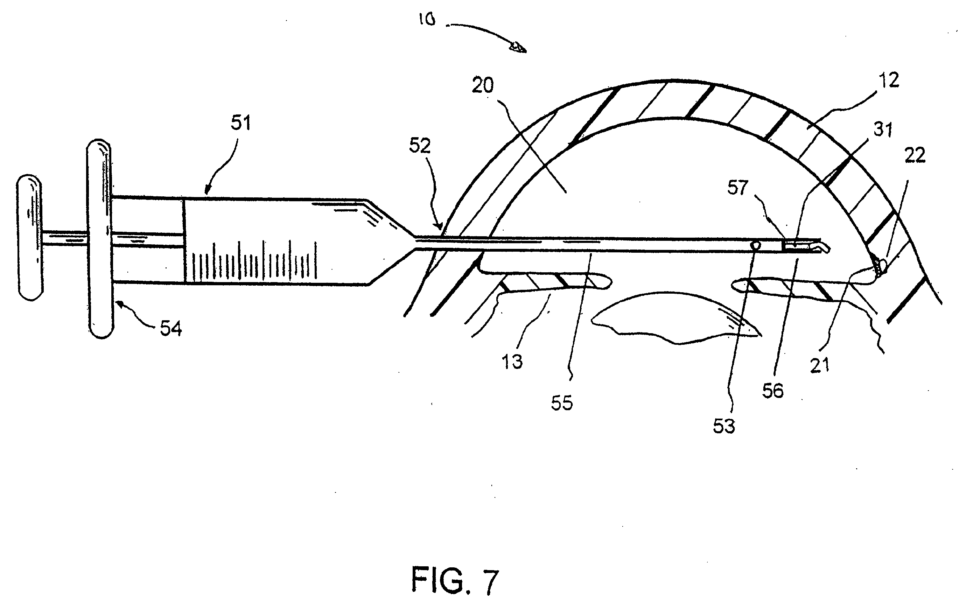

**FIGURE 7** generally illustrates a preferred method by which the trabecular shunting device 31 is implanted within the eye 10. In the illustrated method, a delivery applicator 51 is provided, which preferably comprises a syringe portion 54 and a cannula portion 55 which contains at least one lumen (not shown). The cannula portion 55 preferably has a size of about 30 gauge. However, in other embodiments, the cannula portion 55 may have a size ranging between about 16 gauge and about 40 gauge. A distal section of the cannula portion 55 has at least one irrigating hole 53 in fluid communication with the lumen. A holder for holding the device 31 comprises a lumen 56 having a proximal end 57. In other embodiments, the holder may advantageously comprise a lumen, a sheath, a clamp, tongs, a space, and the like. The proximal end 57 of the lumen 56 is preferably sealed off from the remaining lumen and the irrigating hole 53 of the cannula portion 55. As will be recognized by those skilled in the art, however, in other embodiments of the cannula portion 55, the lumen 56 may advantageously be placed in fluid communication with the lumen and irrigating hole 53 of the cannula portion 55 without detracting from the invention.

In the method illustrated in FIGURE 7, the device 31 is placed into the lumen 56 of the delivery applicator 51 and then advanced to a desired implantation site within the eye 10. The delivery applicator 51 holds the device 31 securely during delivery and releases it when the practitioner initiates deployment of the device 31.

In a preferred embodiment of trabecular meshwork surgery, a patient is placed in a supine position, prepped, draped, and appropriately anesthetized. A small incision 52 is then made through the cornea. The incision 52 preferably has a surface length less than about 1.0 millimeters in length and may advantageously be self-sealing. Through the incision 52, the trabecular meshwork 21 is accessed, wherein an incision is made with an irrigating knife (not shown). The device 31 is then advanced through the corneal incision 52 and across the anterior chamber 20, while the device 31 is held in the delivery applicator 51, under gonioscopic, microscopic, or endoscopic guidance. After the device 31 is appropriately implanted, the applicator 51 is withdrawn and the trabecular meshwork surgery is concluded.

**FIGURE 8** generally illustrates the use of the trabecular shunting device 31 for establishing an outflow pathway, passing from the anterior chamber 20 through the trabecular meshwork 21 to Schlemm's canal 22. As illustrated, an opening has been created in the trabecular meshwork 21. As will be appreciated by those of ordinary skill in the art, such an opening in the trabecular meshwork 21 may comprise an incision made with a microknife, a pointed guidewire, a sharpened applicator, a screw-shaped applicator, an irrigating applicator, a barbed applicator, and the like. Alternatively, the trabecular meshwork 21 may advantageously be dissected with an instrument similar to a retinal pick or microcurrette. Furthermore, the opening may advantageously be created by fiberoptic laser ablation. Referring again to **FIGURE 8**, the outlet section 9 of the device 31 has been inserted in its entirety into the opening in the trabecular meshwork 21. The inlet section 2 is exposed to the anterior chamber 20, while the outlet section 9 is positioned near an interior surface 43 of Schlemm's canal 22. In other embodiments, the outlet section 9 may advantageously be placed into fluid communication with other natural outflow pathways, such as, but not limited to, aqueous collector channels, aqueous veins, and episcleral veins, as described above. A device such as the device 31A of **FIGURE 4**, wherein the outflow section 9A has an open trough 7A for stenting purposes, may be used to maintain an opening of one or more of such natural outflows pathways. With the trabecular shunting device 31 implanted as illustrated in **FIGURE 8**, aqueous flows from the anterior chamber 20 through the device 31 into Schlemm's canal 22, bypassing the trabecular meshwork 21, thereby reducing intraocular pressure within the eye 10.

Although preferred embodiments of the invention have been described in detail, including ab intemo and ab extemo procedures and devices thereof, certain variations and modifications will be apparent to those skilled in the art, including embodiments that do not provide all of the features and benefits described herein. Accordingly, the scope of the present invention is not to be limited by the illustrations or the foregoing descriptions thereof, but rather solely by reference to the appended claims.

The features of the appended claims may be combined in combinations other than those specifically recited.

## Claims

1. A trabecular shunting device that is implantable within an eye, said device comprising:
an inlet section having at least one inlet lumen;
a flow-restricting member within the at least one inlet lumen, said flow-restricting member being configured to prevent at least one component of blood from passing through the flow-restricting member;
an outlet section having a first outlet end and a second outlet end, said outlet section having at least one outlet lumen that opens to at least one of the first and second outlet ends; and
a middle section having at least one middle lumen, said middle section being attached to said outlet section between the first and second outlet ends, said at least one middle lumen being in fluid communication with both said at least one outlet lumen and said at least one inlet lumen;
wherein the device is configured to permit fluid entering said at least one inlet lumen to pass through the flow-restricting member, enter said at least one middle lumen, pass into said at least one outlet lumen, and then exit the outlet section through at least one of said first and second outlet ends.

2. The device of Claim 1, wherein said outlet section is flexible.

3. The device of Claim 1, wherein said middle section is coextensive with said inlet section.

4. The device of Claim 1, wherein said middle section is adjustable in position relative to at least one of said inlet section and said outlet section.

5. The device of Claim 1, wherein said outlet section has a radius of curvature which ranges between about 4 millimeters and about 10 millimeters.

6. The device of Claim 1, wherein a longitudinal axis of the outlet section forms an angle with a longitudinal axis of the middle section, said angle being between about 30 degrees and about 150 degrees.

7. The device of Claim 1, wherein a longitudinal axis of the middle section forms an angle with a longitudinal axis of the inlet section, said angle being between about 20 degrees and about 150 degrees.

8. The device of Claim 1, wherein a junction between said inlet section and said middle section comprises a circumferential ridge, wherein a distance between the circumferential ridge and a junction between the middle section and said outlet section is between about 100 micrometers and about 300 micrometers.

9. The device of Claim 1, wherein said outlet section further comprises at least one side opening that is in fluid communication with said lumen of the outlet section.

10. The device of Claim 1, wherein said outlet section further comprises at least one protuberance that projects from an exterior surface of the outlet section.

11. The device of Claim 1, wherein said outlet section further comprises at least one spur that projects from an exterior surface of the outlet section.

12. The device of Claim 1, wherein said outlet section comprises an elongate trough that is in fluid communication with the lumen of said middle section.

13. The device of Claim 1, wherein said inlet section has an exterior diameter ranging between about 30 micrometers and about 500 micrometers.

14. The device of Claim 1, wherein the lumen within said inlet section has a diameter between about 20 micrometers and about 250 micrometers.

15. The device of Claim 1, wherein said middle section has an exterior diameter between about 30 micrometers and about 500 micrometers.

16. The device of Claim 1, wherein the lumen within said middle section has a diameter between about 20 micrometers and about 250 micrometers.

17. The device of Claim 1, wherein said outlet section has an exterior diameter between about 30 micrometers and about 500 micrometers.

18. The device of Claim 1, wherein the lumen within said outlet section has a diameter between about 20 micrometers and about 250 micrometers.

19. The device of Claim 1, wherein said outlet section has a longitudinal length between about 0.05 centimeters and about 10 centimeters.

20. The device of Claim 1, wherein said outlet section and said lumen within the outlet section have a generally ovoid cross-section.

21. The device of Claim 1, wherein said device is coated with at least one polymer film that contains at least one pharmaceutical substance, said polymer film permitting a delivery of a quantity of the pharmaceutical substance to ocular tissues over time.

22. The device of Claim 21, wherein said delivery is activated by incidence of an electromagnetic field.

23. The device of Claim 22, wherein said electromagnetic field arises due to Nuclear Magnetic Resonance (NMR)

24. The device of Claim 22, wherein said electromagnetic field arises due to Magnetic Resonance Imaging (MRI)

25. The device of Claim 22, wherein said electromagnetic field arises due to short range RF.

26. The device of Claim 21, wherein said delivery is activated by ultrasound waves.

27. The device of Claim 1, wherein said device is made of a material comprising at least one pharmaceutical substance admixed with a polymer substrate.

28. The device of Claim 27, wherein said polymer substrate is selected from the group consisting of poly(lactic acid), polyethylene-vinyl acetate), poly(lactic-co-glycolic acid), poly(D,L-lactide), poly(D,L-lactide-co-trimethylene carbonate), collagen, heparinized collagen, poly(caprolactone), poly(glycolic acid), and copolymer.

29. The device of Claim 1, wherein said device is made of a material comprising at least one pharmaceutical substance admixed with a biodegradable substrate, wherein said biodegradable substrate is selected from the group consisting of poly(lactic acid), polyethylene-vinyl acetate, poly(lactic-co-glycolic acid), poly(D,L-lactide), poly(D,L-lactide-co-trimethylene carbonate), collagen, heparinized collagen, poly(caprolactone), poly(glycolic acid), and copolymer.

30. An apparatus for implanting a trabecular shunting device within an eye, said apparatus comprising:
a syringe portion;
a cannula portion having proximal and distal ends, said distal end of said cannula portion attached to said syringe portion, said cannula portion further comprising a first lumen and at least one irrigating hole, said hole disposed between said proximal and distal ends of the cannula portion, said irrigating hole being in fluid communication with the lumen; and
a holder comprising a second lumen for holding the trabecular shunting device, wherein a distal end of the second lumen opens to said distal end of the cannula portion, and a proximal end of the second lumen is separated from said first lumen of the cannula portion;
wherein said holder holds the trabecular shunting device during implantation of said device within the eye, and said holder releases the trabecular shunting device when a practitioner activates deployment of said device.

31. The apparatus of Claim 30, wherein said cannula portion has a size ranging between about 16 gauge and about 40 gauge.

32. The apparatus of Claim 30, wherein said cannula portion has a size of about 30 gauge.

33. The apparatus of Claim 30, wherein said cannula portion has at least one lumen which is in fluid communication with said irrigating hole and with said holder.
